# EUROPÄISCHE PATENTSCHRIFT

(11) **EP 2 424 477 B1**
(45) Veröffentlichungstag und Bekanntmachung des Hinweises auf die Patenterteilung: **15.08.2018**
(21) Anmeldenummer: 10716300.8
(22) Anmeldetag: 27.04.2010
(51) Int. Cl.: A61M 1/00, A61F 13/00, A61F 13/02

(54) **FOLIE FÜR WUNDABDECKUNG**
FILM FOR COVERING A WOUND
FEUILLE POUR PANSEMENT

(30) Priorität: 30.04.2009 DE 102009019646
(43) Veröffentlichungstag der Anmeldung: 07.03.2012
(60) Teilanmeldung: 17198606.0
(73) Patentinhaber: Lohmann & Rauscher GmbH, 2525 Schönau/Triesting (AT)
(72) Erfinder: WILD, Thomas, 1200 Wien (AT); WAGNER, Georg, 4040 Linz (AT); ROHRER, Christian, 4020 Linz (AT); STEINLECHNER, Erik, 2500 Baden (AT)
(74) Vertreter: Seranski, Klaus
(86) Internationale Anmeldenummer: PCT/EP2010/002576
(87) Internationale Veröffentlichungsnummer: WO 2010/124844

(56) Entgegenhaltungen:
- DE-A1-102006 017 194
- DE-A1-102007 049 428
- DE-A1-102008 034 362
- DE-U1-202006 015 547
- US-A- 4 341 217
- US-A1- 2004 030 304

## Beschreibung

Die Erfindung betrifft eine Wundabdeckung mit einem eine erste Begrenzungsfläche bildenden ersten bahnförmigen Element und einem eine der ersten Begrenzungsfläche abgewandte und etwa parallel dazu verlaufende zweite Begrenzungsfläche bildenden und mit dem ersten bahnförmigen Element verbundenen zweiten bahnförmigen Element, sowie ein eine derartige Wundabdeckung aufweisendes Wundversorgungskit und ein Verfahren zum Herstellen erfindungsgemäßer Wundabdeckungen.

Derartige Wundabdeckungen werden bei verschiedenartigen Krankheitsbildern bzw. Traumata, insbes. im Bereich des offenen Abdomens benötigt, wenn eine temporäre Abdeckung der offenen Wunde bzw.. des offenen Abdomens notwendig ist. Das kann bspw. notwendig sein, wenn mehrere Eingriffe täglich erforderlich sind, um einerseits einen schnellen Zugang zu den inneren Organen zu ermöglichen und andererseits einen nachteilhaften Einfluß der Exsudatbildung im Wundbereich zu vermeiden. Durch eine temporäre Abdeckung kann ein deutlicher Rückgang der Mortalität bei einigen Indikationen erreicht werden.

An für die genannten Einsatzzwecke geeignete Wundabdeckungen werden grundsätzlich aus medizinischer Sicht zwei Hauptanforderungen gestellt. Zum einen muß ein gutes Exsudatmanagement im Wundbereich, insbes. bei Einsatz im offenen Abdomen, erreicht werden, d. h. eine Absaugung im gesamten Wundbereich bzw. im gesamten Abdomen. Darüber hinaus sollte eine Friktionsminderung zwischen Organen und Bauchfell und der Wundabdeckung erreicht werden, während gleichzeitig die offene Wunde von der Umwelt gut abgeschirmt ist. Darüber hinaus muß sichergestellt werden, daß durch die Wundabdeckung keine Verunreinigungen in die offene Wunde bzw. den offenen Abdominalbereich gelangen.

In der EP 0 261 167 B1 ist eine flüssigkeitsdurchlässige und für den direkten Kontakt mit dem Wundgrund vorgesehene Wundabdeckung beschrieben, welche eine hydrophobe Schicht aufweist, um das Anhaften der Wundabdeckung am Wundbereich und eine dadurch ggf. verursachte Verunreinigung der Wunde zu verhindern. Mit der in dieser Schrift beschriebenen Wundabdeckung kann jedoch kein zufriedenstellendes Exsudatmanagement im Wundbereich erhalten werden.

Zur Verbesserung des Exsudatmanagements im Wundbereich wird in der US 7,381,859 B2 eine Wundabdeckung vorgeschlagen, bei der zwischen zwei flüssigkeitsdurchlässigen, folienartigen, bahnförmigen Elementen, die in Form von Kunststofffilmen ausgeführt sein können, eine schaumartige Schicht aufgenommen ist, in der Exsudate absorbiert werden können. Bei der aus dieser Schrift bekannten Wundabdeckung hat es sich jedoch als problematisch erwiesen, daß in Randbereichen der Wundabdeckung keine zufriedenstellende Absaugung von Exsudaten erfolgen kann, so daß es in diesen Randbereichen zu Komplikationen bei der Wundversorgung kommt.

In der WO 2007/118652 A1 ist ein Wunddistanzgitter beschrieben, auf das saugfähige Sekundärverbände kleblos auflegbar sind und welches zur Bildung einer ersten glatten Oberfläche und einer zweiten Oberfläche mit einem rauhen Griff mit einer Vielzahl von dreidimensionalen Perforationen ausgestattet ist. Bei Einsatz dieser bekannten Wundabdeckung sind die nachgeschalteten Absorptionskörper auswechselbar, um so über einen längeren Zeitraum eine zufriedenstellende Wundversorgung zu gewährleisten. Allerdings hat es sich auch bei Einsatz der in der genannten Schrift beschriebenen Wundabdeckungssysteme als problematisch erwiesen, daß ein zufriedenstellendes Exsudatmanagement über den gesamten Wundbereich, insbes. im Abdominalbereich, nicht erreichbar ist.

In der DE 10 2008 034 362 A1 ist ein Wundpflegeartikel zum Aufsaugen von Wundflüssigkeit beschrieben, der einen Materialabschnitt aus einem Absorptionsmaterial mit hoch hydroaktiven Polymeren sowie mindestens eine flüssigkeitsdurchlässige Kaschierung aufweist, die unmittelbar an dem flachen Materialabschnitt anliegt und den Durchtritt von an der Wunde austretenden flüssigen Substanzen zu dem Materialabschnitt aus Absorptionsmaterial ermöglicht.

Die DE 10 2006 017 194 A1 offenbart die Verwendung eines flüssigkeitsdurchlässigen schmiegsamen Materialabschnitts aus einem Thermoplasten, der eine erste, glatte Oberfläche sowie eine der glatten Oberfläche abgewandte zweite Oberfläche des Materialabschnitts aufweist, wobei eine Vielzahl von dreidimensionalen Perforationen vorgesehen ist, deren Wände von der ersten, glatten Oberfläche beginnend, jeweils in einen Randüberstand mit freier Kante auslaufen und der zweiten Oberfläche einen rauhen Griff verleihen, zur Wundabdeckung als sterilisierter Primärverband, auf den saugfähige Sekundärverbände auflegbar sind und der den Verklebungen und Adhäsionen mit dem Exsudat entgegenwirkt.

Die DE 20 2006 015 547 U1 offenbart eine Stützkompresse, die eine Vliesmatte mit superabsorbenden Teilchen aufweist und der wenigstens eine Schaumstoffmatte zugeordnet ist.

Die DE 10 2007 049 428 A1 betrifft einen Wundpflegeartikel mit einer superabsorbierende Polymere aufweisenden Lage sowie einer Lage mit einem Weichschaumstoff.

Die US 2004/030304 beschreibt ein System und ein Verfahren zur temporären Versorgung einer Wunde, insbesondere einer Abdominalwunde, mit der der Heilungsvorgang der Wunde begünstigt wird, wobei ein Sauganschluß auf einer oberen Begrenzungsfläche einer Schaumstoffschicht vorgesehen ist, um einen Unterdruck an die Wunde anzulegen.

In der US 341,217 ist eine Damenbinde mit einem eine erste Begrenzungsfläche bildenden und mit den Durchtritt von Körperflüssigkeiten in den Drainageraum ermöglichenden Perforationen versehenen bahnförmigen Element, einem zweiten mit entsprechenden Perforationen ausgestatteten bahnförmigen Element und einem zwischen den bahnförmigen Elementen angeordneten Absorptionskörper beschrieben.

Angesichts der vorstehend beschriebenen Probleme im Stand der Technik liegt der Erfindung die Aufgabe zugrunde, eine Wunderversorgungskit bereitzustellen, mit der ohne Verunreinigung der Wunde selbst ein gutes Exsudatmanagement über den gesamten Wundbereich möglich ist.

Erfindungsgemäß wird diese Aufgabe durch eine Wundabdeckung gemäß Patentanspruch 1 gelöst.

Bei der erfindungsgemäß eingesetzten Wundabdeckung wird durch die Bildung des Drainageraums eine kapillare Wirkung zwischen den einzelnen bahnförmigen Elementen erreicht, mit der einerseits ein Abfließen von Körperflüssigkeiten, insbes. Exsudaten, aus dem Drainageraum verhindert und andererseits eine Verteilung der Körperflüssigkeiten über die gesamte Drainagefläche möglich ist, ohne daß dazu zusätzliche Maßnahmen, wie etwa die Bereitstellung zusätzlicher Absorptionskörper, erforderlich sind. Auf diese Weise wird ein gutes Exsudatmanagement über den gesamten Wundbereich ermöglicht, weil auch keine das Exsudatmanagement nachteilhaft beeinflussenden Randbereiche ohne Kapillarfunktion erforderlich sind. Die erfindungsgemäße Wundabdeckung läßt sich problemfrei auf jede Größe bzw. jede Struktur von Wunden, im besonderen im Bauchraum, zuschneiden und gewährleistet über die gesamte Wundabdeckungsfläche die gewünschte Drainagefunktion. Dabei kann die Wundabdeckung auch mit einer glatten Oberfläche ausgeführt sein, die ein Anhaften von Geweben und Zellwundgrund bzw. bei den umliegenden Organen verhindert. Der Eintritt von Körperflüssigkeiten in den Drainageraum kann bei Einsatz erfindungsgemäßer Wundabdeckungen bereits dadurch sichergestellt werden, daß die bahnförmigen Elemente selbst aus einem flüssigkeitsdurchlässigen Material gebildet sind.

Im Rahmen der Erfindung hat es sich jedoch als besonders günstig erwiesen, wenn mindestens eines der bahnförmigen Elemente eine den Durchtritt von Körperflüssigkeiten in den Drainageraum ermöglichende Öffnung aufweist. Dabei kann die Stabilität der Gesamtstruktur erfindungsgemäßer Wundabdeckungen verbessert werden, wenn mindestens eine Öffnung durch einen sich ausgehend von einem bahnförmigen Element in Richtung auf die gegenüberliegende innere Begrenzungsfläche des anderen bahnförmigen Elements erstreckenden und in den Drainageraum mündenden Kanal gebildet ist, dessen Kanalwand vorzugsweise einstückig mit dem bahnförmigen Element ausgeführt, insbes. durch Perforation des bahnförmigen Elements, gebildet ist.

Bei der Erfindung wird das Exsudatmanagement besonders wirkungsvoll verbessert weil sich die Querschnittsfläche des Kanals in einer sich senkrecht zur Tiefenrichtung erstreckenden Ebene ausgehend von dem bahnförmigen Element in Richtung auf die gegenüberliegende innere Begrenzungsfläche, insbes. zum Erhalt einer den Eintritt von Körperflüssigkeiten in den Drainageraum begünstigenden kapillaren Wirkung, verringert. Auf diese Art und Weise wird einerseits der Abtransport von Exsudat aus dem Wundbereich unterstützt und andererseits ein Zurückfließen aus dem Drainageraum in den Wundraum verhindert.

Bei erfindungsgemäßen Wundabdeckungen wird der gewünschte Abtransport des Exsudats unter Sicherstellung einer zufriedenstellenden Gesamtstabilität der Wundabdekkung erreicht, weil mindestens ein bahnförmiges Element eine Vielzahl von vorzugsweise rasterförmig angeordneten, insbes. in einem Rechteckraster angeordneten Öffnungen, aufweist, wobei der Abstand zwischen benachbarten Öffnungen bzw. Rasterpunkten 15 mm oder weniger, vorzugsweise 5 mm oder weniger, insbes. 3 mm oder weniger, beträgt.

Im Hinblick auf die gewünschte Gesamtstabilität erfindungsgemäßer Wundabdekkungen hat es sich weiter als besonders zweckmäßig erwiesen, dass die Mündungen der in einem der bahnförmigen Elemente angeordneten Öffnungen in einer Projektion längs der Tiefenrichtung zwischen den Mündungen der in dem anderen bahnförmigen Element angeordneten Öffnungen angeordnet sind.

Diese Anordnung der Öffnungen in erfindungsgemäßen Wundabdeckungen wird mit besonderem Vorteil bei durch Kanäle gebildeten Öffnungen eingesetzt, weil die Wundabdeckung in Verbindung mit einer zum Absaugen des Exsudats aus dem Wundbereich, insbes. Bauchraum, eingesetzten Unterdruckquelle verwendet wird, um so ein Kollabieren der Gesamtstruktur bei Anlegen von Unterdruck zu verhindern. In diesem Zusammenhang hat es sich als besonders günstig erwiesen, dass sich mindestens ein eine Öffnung in einem bahnförmigen Element bildender Kanal in Tiefenrichtung über 50 % oder mehr der gesamten Tiefe des Drainageraums erstreckt.

Zur Sicherstellung der gewünschten Durchlässigkeit der bahnförmigen Elemente hat es sich als zweckmäßig erwiesen, dass die dem Drainageraum zugewandte Mündungsfläche der einzelnen Öffnungen in einer senkrecht zur Tiefenrichtung verlaufenden Ebene 0,1 mm² oder mehr, insbes. 0,5 mm² oder mehr, besonders bevorzugt 1 mm² oder mehr, beträgt. Die gewünschte Kapillarwirkung wird unter Vermeidung eines Rückflusses von Flüssigkeit aus dem Drainageraum in den Wundraum erreicht, weil die Mündungsfläche der Öffnungen 5 mm² oder weniger, insbes. 4 mm² oder weniger, besonders bevorzugt 3 mm² oder weniger, beträgt.

Wie vorstehend bereits erläutert, können die die Öffnungen in den bahnförmige Elemente bildenden Kanäle durch Perforationen in den bahnförmigen Elementen gebildet sein. In diesem Zusammenhang hat es sich zum Erhalt einer glatten Oberfläche, mit der ein Anhaften von Gewebe bzw. Zellen am Wundgrund bzw. bei den umliegenden Organen wirkungsvoll unterdrückt werden kann, als günstig erwiesen, dass die Kanalwand in einer sich parallel zur Tiefenrichtung erstreckenden Schnittebene zumindest abschnittweise bogenförmig ausgeführt ist und stetig in die Begrenzungsfläche des bahnförmigen Elements übergeht. Die Verbindung der bahnförmigen Elemente kann unter gleichzeitiger Sicherstellung des die Drainagewirkung hervorbringenden Drainageraums über punktförmige und vorzugsweise in einer Rasteranordnung ausgebildete Befestigungsbereiche erfolgen. Die Befestigung kann dabei mittels Schweißen, Kleben oder anderen festen Verbindungsarten geschehen. Dabei sollte durch die Verbindung der Abtransport von Exsudaten jedoch nicht behindert, sondern unterstützt werden. In diesem Zusammenhang hat es sich als günstig erwiesen, wenn die einzelnen Befestigungsbereiche in einer sich senkrecht zur Tiefenrichtung erstreckenden Schnittebene eine Fläche von 5 mm² oder weniger, insbes. 3 mm² oder weniger, besonders bevorzugt 2 mm² oder weniger, aufweisen, wobei der Abstand zwischen einzelnen Befestigungsbereichen bzw. einzelnen Rasterpunkten der in einer Rasteranordnung ausgeführten Befestigungsbereiche 2 mm oder mehr, im besonderen 3 mm oder mehr, besonders bevorzugt 5 mm oder mehr, beträgt.

- Unabhängig davon, ob die Befestigung durch Schweißen, Kleben oder andere Verbindungsarten erfolgt, kann in den Befestigungsbereichen eine die inneren Begrenzungsflächen der bahnförmigen Elemente miteinander verbindenden Materialbrücke gebildet sein. Zum Erhalt der gewünschten Kapillarwirkung im Drainageraum beträgt der Abstand zwischen den inneren Begrenzungsflächen der bahnförmigen Elemente bei erfindungsgemäßen Wundabdeckungen 5 mm oder weniger, vorzugsweise 4 mm oder weniger, besonders bevorzugt 2 mm oder weniger. Dabei wird die Verteilung der Exsudate über die Gesamtfläche der Wundabdeckung unter Ausnutzung der Kapillarwirkung im Drainageraum sichergestelt, weil der Abstand zwischen den inneren Begrenzungsflächen der bahnförmigen Elemente 0,05 mm oder mehr, insbes. 0,1 mm oder mehr, besonders bevorzugt 0,3 mm oder mehr, beträgt.

Bei erfindungsgemäßen Wundabdeckungen weist mindestens ein bahnförmiges Element eine vorzugsweise perforierte Kunststofffolie auf, und kann insbes. insgesamt in Form einer perforierten Kunststofffolie gebildet sein.

Die erfindungsgemäße Wundabdeckung ist mit besonderem Vorteil in Kombination mit der Unterdrucktherapie zur Wundversorgung einsetzbar. Dabei wird die erfindungsgemäße Wundabdeckung als Wundauflage bzw. als direktes Interface zwischen dem Wundgrund (Gewebe, Zellen) und dem Wundfüller bzw. dem Bereich des Exsudatabtransports eingesetzt. Mit besonderem Vorteil ist die erfindungsgemäße Wundabdeckung für den Einsatz bei Wunden im offenen Abdomen geeignet. Dabei wird die erfindungsgemäße Wundabdeckung in dem gesamten Bauchraum über die Organe (Eingeweide) gelegt, wobei die Wundabdeckung eine Drainagefunktion und eine sehr glatte Oberfläche bietet, um ein Anhaften bzw. eine Friktion von Gewebe und Folie zu verhindern. Die erfindungsgemäße Wundabdeckung kann anatomisch vorgeformt, aber auch auf spezielle Gegebenheiten zuschneidbar ausgeführt sein. Auf die erfindungsgemäße Wundabdeckung wird bei Einsatz in Kombination mit der Unterdrucktherapie ein Füllmedium wie Gaze oder Schaum aufgefüllt, bis der Bereich bis zur Hautgrenze abgedeckt ist. In diesen Wundfüller kann eine ggf. schlauchförmige Wunddrainage eingelegt werden. Der zur Ableitung der Exsudate bzw. zur Wunddrainage eingesetzte Schlauch wird aus dem Bauchraum herausgeführt und an eine Unterdruckquelle angeschlossen, die durch eine Pumpe bzw. im Krankenhaus durch eine zentrale Hausversorgung sichergestellt werden kann. Abschließend wird die Wunde okklusiv mit einer adhäsiven Folie abgedichtet. Mit der Unterdruckquelle kann dann das Exsudat aus dem gesamten Bauchraum mittels der erfindungsgemäßen Wundabdeckung abtransportiert werden, wodurch ein gutes Exsudatmanagement erreicht wird. Die Folie für die okklusive Abdeckung der Wunde ist für die Abschirmung der inneren Organe von der Außenwelt verantwortlich und regelt Feuchtigkeit und Temperatur. Ferner ist sie eine mit der Haut vergleichbare Barriere gegen Bakterien, Viren und Keime.

Wie der vorstehenden Erläuterung zu entnehmen ist, weist ein erfindungsgemäßes Wundversorgungskit eine erfindungsgemäße Wundabdeckung, einen darauf angeordneten Absorptionskörper, wie etwa einen Schaumkörper oder einen Gazekörper, eine okklusive Folie und ein schlauchförmiges Ableitungselement auf, das an eine Unterdruckquelle angeschlossen werden kann. Die Herstellung erfindungsgemäßer Wundabdeckungen besteht im wesentlichen daraus, daß ein bahnförmiges Material, bspw. unter Verwendung einer Stanzwalze, zur Bildung trichterförmiger Öffnungen perforiert wird, zwei perforierte Abschnitte des bahnförmigen Materials mit einander zugewandten Trichteröffnungen übereinandergelegt und anschließend bspw. mittels eines Punktschweißverfahrens miteinander verbunden werden. Bei diesem Verfahren kann der gewünschte Abstand zwischen den inneren Begrenzungsflächen durch die Tiefer der trichterförmigen Öffnungen sichergestellt werden. Dabei kann der Perforationsvorgang allerdings nur mit einer Genauigkeit erfolgen, so daß selbst dann, wenn einige Trichteröffnungen an der gegenüberliegenden inneren Begrenzungsfläche des gegenüberliegenden bahnförmigen Elements anliegen, andere Trichteröffnungen noch frei in den Drainageraum münden.

Im Hinblick auf die Vermeidung der Verklebungsneigung erfindungsgemäßer Wundabdeckungen kann mindestens eine Materialbahn eine hydrophile oder hydrophobe Ausrüstung aufweisen. Ferner ist auch an den Auftrag von quellbaren Materialien gedacht.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: eine schematische Darstellung einer erfindungsgemäßen Wundabdeckung und
- **Fig. 2**: ein Anwendungsbeispiel eines erfindungsgemäßen Wundversorgungskits

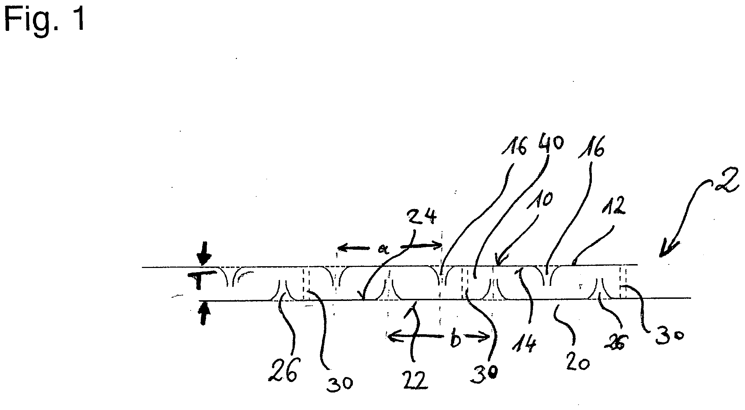

Die in Fig. 1 dargestellte Wundabdeckung umfaßt ein erstes, eine äußere Begrenzungsfläche 12 aufweisendes, bahnförmiges Element sowie ein zweites, eine äußere Begrenzungsfläche 22 aufweisendes, bahnförmiges Element 20. Zwischen einer dem zweiten bahnförmigen Element 20 zugewandten inneren Begrenzungsfläche 14 des ersten bahnförmigen Elements 10 und einer dem ersten bahnförmigen Element 10 zugewandten inneren Begrenzungsfläche 24 des zweiten bahnförmigen Elements 20 ist ein Drainageraum 40 gebildet. Der Tiefe des Drainageraums 40 in einer senkrecht zu den Hauptflächen der bahnförmigen Elemente 10 und 20 verlaufenden Richtung beträgt bei der in der Zeichnung dargestellten Ausführungsform der Erfindung zwischen 0,1 und 4 mm.

Das erste bahnförmige Element 10 weist eine Mehrzahl von rasterförmig angeordneten, kanalförmigen Perforationen 16 auf. Die Kanalwände der Kanäle 16 sind in einer parallel zur Tiefenrichtung verlaufenden Schnittebene bogenförmig ausgeführt und gehen glatt in die äußere Begrenzungsfläche 12 des ersten bahnförmigen Elements 10 über. Ebenso weist das zweite bahnförmige Element 20 eine Anzahl von rasterförmig angeordneten, ebenfalls kanalförmigen Perforationen 26 auf. Die Kanäle 16 und 26 erstrecken sich jeweils über mehr als die Hälfte der Gesamttiefe T des Drainageraums 40. Dabei sind die Kanäle 12 und 16 derart angeordnet, daß eine Projektion der Kanalmündungen der Kanäle 16 in der Tiefenrichtung zwischen den Kanalmündungen der Kanäle 26 zu liegen kommt.

Im Mündungsbereich weisen die bei der anhand der Zeichnung erläuterten Ausführungsform der Erfindung kegelförmig ausgeführten Kanäle 16 bzw. 26 einen Durchmesser von 0,2 bis 1 mm auf. Die bahnförmigen Elemente 10 und 20 sind über rasterförmig angeordnete Materialbrücken 30 miteinander verbunden. Durch die Materialbrücken 30 wird der Abstand zwischen den inneren Begrenzungsflächen 14 und 24 eingestellt. Die Verbindungsbereiche 30 können durch Schweißen, Kleben oder andere feste Verbindungsarten verwirklicht werden. Die bahnförmigen Elemente 10 und 20 können in Form von Kunststofffolien verwirklicht werden. In diesem Zusammenhang können sowohl synthetische Kunststoffe, wie etwa PE, PP, PET, Polyurethan (PUR), Teflon (PTFE), als auch Folien auf Basis biologischer Kunststoffe bzw. nachwachsender Kunststoffe, wie etwa Polyhydroxybutyrat (PHB), Polylactat (PLA), Polymilchsäure, Rohstoffe auf Basis von Cellulose (CMC) usw. zum Einsatz kommen. Die Struktur der perforierten Materialbahnen kann mittels eines non woven Herstellungsprozesses erzeugt werden. Dabei können zur Herstellung der non wovens oder auch Gewebe synthetische wie auch natürliche (z. B. Seide, Baumwolle) Fasern, wie auch anorganische Fasern (Glaskeramik...) bzw. Metall (Silberfasern) verwendet werden. Im Rahmen der Erfindung ist ferner daran gedacht, daß die Materialbahnen 10, 20 bzw. der Drainageraum 40 mit einer antibakteriellen bzw. bakteriostatischen Schicht ausgestattet werden. Die antibakterielle bzw. bakertiostatische Wirkung kann bspw. durch PHMB, Silber, Chlorhexidin usw. verwirklicht werden.

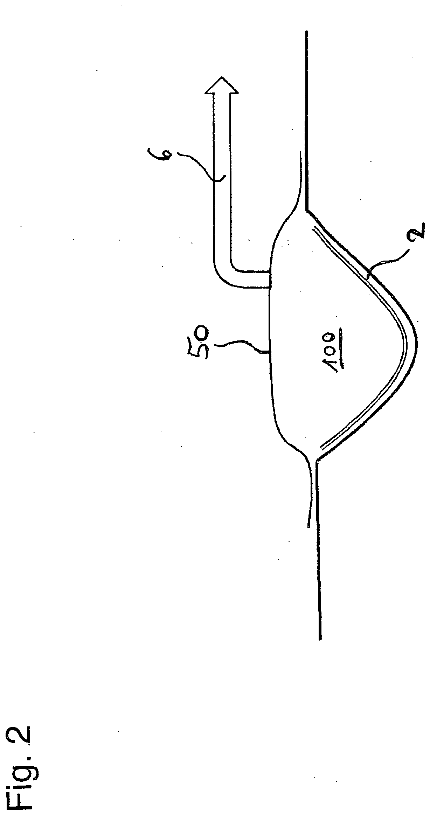

Gemäß dem anhand der Figur 2 erläuterten Anwendungsbeispiel wird die Wundabdeckung 2 auf dem Wundgrund aufgelegt, die Wunde danach mit einem Füllmaterial, wie etwa einem Schaum, Gaze od. dgl., aufgefüllt und mit einer okklusiven Folie 50 abgedeckt. Die okklusive Folie 50 wird von einer Exsudatleitung 6 durchsetzt, welche zum Absaugen des Exsudats aus dem Wundraum an eine Unterdruckquelle angeschlossen werden kann.

Bei Einsatz erfindungsgemäßer Wundabdeckungen ergeben sich, wie vorstehend erläutert, die folgenden Vorteile:
- Die Poren mit deren nach innen sich erhebenden Struktur unterstützen den Abtransport von Exsudat und verhindern das Zurückfließen des Exsudats in den Wundraum (Kapillarwirkung).
- Die offenen Drainagekammern geben der Drainageschicht 40 eine kapillare Wirkung und lassen somit kein Abfließen aus der Wundabdeckung zu.
- Die durch den glatten Übergang zwischen den Kanälen 16 bzw. 26 und den äußeren Begrenzungsflächen 12 bzw. 24 der bahnförmigen Elemente erhaltene glatte Oberfläche verhindert ein Anhaften von Gewebe bzw. Zellen am Wundgrund bzw. bei den umliegenden Organen.
- Die Kombination aus Porengröße und Glattheit der Folie verhindert ein Einwachsen bzw. Anhaften von Gewebe.
- Der Drainageraum 40 bietet immer einen offenen Raum für den Abtransport von Exsudat.
- Die Wundabdeckung ist ohne Beeinträchtigung der Drainagefunktion auf jede Größe bzw. Struktur im Wund- bzw. Abdominalraum zuschneidbar.

Abschließend wird darauf hingewiesen, daß der Abstand zwischen den Zentrumsachsen der Kanäle 16 bzw. 26, wie in Figur 1 bei a und b angedeutet, der anhand der Zeichnung erläuterten Ausführungsform der Erfindung mindestens 0,1 mm und maximal 4 mm beträgt.

Die Erfindung ist nicht auf das anhand der Zeichnung erläuterte Ausführungsbeispiel beschränkt. Auch Wundabdeckungen mit einem mehrschichtigen Aufbau zur Bildung von zwei, drei oder mehr übereinander angeordneten Drainageräumen denkbar. In diesem Fall bilden die den inneren Begrenzungsflächen abgewandten Begrenzungsflächen der bahnförmigen Elemente zumindest bei den innen liegenden Materialbahnen keine äußeren Begrenzungsflächen.

## Patentansprüche

1. Wundabdeckung für den Einsatz in Kombination mit der Unterdrucktherapie bei Wunden im offenen Abdomen mit einem eine erste Begrenzungsfläche (12) bildenden ersten bahnförmigen Element (10) und einem eine der ersten Begrenzungsfläche (12) abgewandte und etwa parallel dazu verlaufende zweite Begrenzungsfläche (22) bildenden und mit dem ersten bahnförmigen Element (10) verbundenen zweiten bahnförmigen Element (20), wobei zwischen der der ersten Begrenzungsfläche (12) abgewandten ersten inneren Begrenzungsfläche (14) des ersten bahnförmigen Elements (10) und einer der zweiten Begrenzungsfläche (22) abgewandten zweiten inneren Begrenzungsfläche (24) des zweiten bahnförmigen Elements (20) mindestens ein offener Drainageraum (40) ohne zusätzlichen Absorptionskörper darin gebildet ist, wobei die Tiefe (T) des Drainageraums in einer sich senkrecht zu den Begrenzungsflächen (12, 22) erstreckenden Tiefenrichtung zur Gewährleistung einer Kapillarwirkung auf in dem Drainageraum (40) aufgenommene Körperflüssigkeiten, insbesondere Exsudate, 5 mm oder weniger, insbesondere 4 mm oder weniger, besonders bevorzugt 2 mm oder weniger beträgt, mindestens eines der bahnförmigen Elemente (10, 20) eine den Durchtritt von Körperflüssigkeiten in den Drainageraum (40) ermöglichende Öffnung aufweist, mindestens eine Öffnung durch einen sich ausgehend von einem bahnförmigen Element (10, 20) in Richtung auf die gegenüberliegende innere Begrenzungsfläche (14, 24) des anderen bahnförmigen Elements (10, 20) erstreckenden und in den Drainageraum (40) mündenden Kanal (16, 26) gebildet ist, dessen Kanalwand vorzugsweise einstückig mit dem bahnförmigen Element (10, 20) ausgeführt, insbes. durch Perforation des bahnförmigen Elements (10, 20), gebildet ist, sich die Querschnittsfläche des Kanals (16, 26) in einer sich senkrecht zur Tiefenrichtung erstreckenden Ebene ausgehend von dem bahnförmigen Element (10, 20) in Richtung auf die gegenüberliegende andere Begrenzungsfläche (14, 24), insbes. zum Erhalt einer den Eintritt von Körperflüssigkeiten in den Drainageraum (40) begünstigenden Kapillarwirkung, verringert, mindestens ein bahnförmiges Element (10, 20) eine Vielzahl von vorzugsweise rasterförmig angeordneten Öffnungen aufweist, wobei der Abstand zwischen benachbarten Öffnungen 15 mm oder weniger, vorzugsweise 5 mm oder weniger, insbes. 3 mm oder weniger, beträgt, die Mündungen der in einem bahnförmigen Element (10, 20) angeordneten Öffnungen in einer Projektion längs der Tiefenrichtung zwischen den Mündungen der in dem anderen bahnförmigen Element (10, 20) angeordneten Öffnungen angeordnet sind, sich mindestens ein Kanal (16, 26) in Tiefenrichtung über 50 % oder mehr der gesamten Tiefe (T) des Drainageraums (40) erstreckt, die dem Drainageraum (40) zugewandte Mündungsfläche der einzelnen Öffnungen in einer senkrecht zur Tiefenrichtung verlaufenden Ebene 0,5 mm² oder mehr, besonders bevorzugt 1 mm² oder mehr, beträgt, die dem Drainageraum (40) zugewandte Mündungsfläche der einzelnen Öffnungen in einer senkrecht zur Tiefenrichtung verlaufenden Ebene 5 mm² oder weniger, insbes. 4 mm² oder weniger, besonders bevorzugt 3 mm² oder weniger, beträgt, die Kanalwand in einer sich parallel zur Tiefenrichtung erstreckenden Schnittebene zumindest abschnittweise bogenförmig ausgeführt ist und stetig in die Begrenzungsfläche (12, 22) übergeht, der Abstand zwischen den inneren Begrenzungsflächen (14, 24) 0,05 mm oder mehr, insbes. 0,1 mm oder mehr, besonders bevorzugt 0,3 mm oder mehr, beträgt und mindestens ein bahnförmiges Element (10, 20) eine vorzugsweise perforierte Kunststofffolie aufweist.

2. Wundabdeckung nach Anspruch 1, **dadurch gekennzeichnet, daß** die bahnförmigen Elemente (10, 20) über punktförmige und vorzugsweise in einer Rasteranordnung ausgeführte Befestigungsbereiche (30) miteinander verbunden sind.

3. Wundabdeckung nach einem der vorhergehenden Ansprüche, **dadurch gekennzeichnet, daß** die einzelnen Befestigungsbereiche (30) in einer sich senkrecht zur Tiefenrichtung erstreckenden Schnittebene eine Fläche von 5 mm² oder weniger, insbes. 3 mm² oder weniger, besonders bevorzugt 2 mm² oder weniger, aufweisen.

4. Wundabdeckung nach Anspruch 2 oder 3 **dadurch gekennzeichnet, daß** in den Befestigungsbereichen (30) eine die inneren Begrenzungsflächen (14, 24) der Materialbahnen (10, 20) verbindende Materialbrücke gebildet ist.

5. Wundabdeckung nach einem der vorhergehenden Ansprüche, **dadurch gekennzeichnet, daß** mindestens ein bahnförmiges Element (10, 20) ein antibakterielles bzw. bakteriostatisches Material, wie etwa PHMB, Silber, oder Chlorhexidin, insbes. in Form einer Oberflächenschicht, aufweist.

6. Wundabdeckung nach einem der vorhergehenden Ansprüche, **dadurch gekennzeichnet, daß** mindestens ein bahnförmiges Element ein der Verklebungsneigung der Wundabdeckung entgegenwirkendes Mittel, wie etwa eine hydrophile oder hydrophobe Ausrüstung oder den Auftrag von quellbaren Materialien aufweist.

7. Wundversorgungskit mit einer Wundabdeckung nach einem der Ansprüche 1 bis 6, einem darauf angeordneten Absorptionskörper einer okklusiven Folie (50) und einer die okklusive Folie durchsetzenden Exsudatleitung (6).

## Claims

1. Wound covering for use in combination with vacuum therapy in wounds in the open abdomen, comprising: a first strip-like element (10) forming a first boundary surface (12) and a second strip-like element (20) forming a second boundary surface (22) facing away from the first boundary surface (12) and extending substantially parallel thereto and connected to the first strip-like element (10); wherein at least one open drainage space (40) free from additional absorbent bodies is formed between the first inner boundary surface (14) of the first strip-like element (10) facing away from the first boundary surface (12) and a second inner boundary surface (24) of the second strip-like element (20) facing away from the second boundary surface (22); wherein the depth (T) of the drainage space, in a depth direction extending perpendicular to the boundary surfaces (12, 22), is 5mm or less, preferably 4mm or less, more preferably 2mm or less, to ensure a capillary effect on body fluids, in particular exudates, received in the drainage space (40); at least one of the strip-like elements (10, 20) has an opening for the passage of body fluids into the drainage space (40); at least one opening is formed by a channel (16, 26) extending from a strip-like element (10, 20) in the direction of the opposite inner boundary surface (14, 24) of the other strip-like element (10, 20) and opening into the drainage space (40), the channel wall of which is preferably formed integrally with the strip-like element (10, 20), in particular by perforation of the strip-like element (10, 20); the cross-sectional area of the channel (16, 26) narrows in a plane extending perpendicular to the depth direction starting from the strip-like element (10, 20) in the direction of the opposite inner boundary surface (14, 24), in particular to obtain a capillary effect favouring the entry of body fluids into the drainage space (40); at least one strip-like element (10, 20) has a plurality of preferably grid-shaped openings, the distance between adjacent openings being 15mm or less, preferably 5 mm or less, more preferably 3mm or less; the mouths of the openings arranged in one strip-like element (10, 20) are situated in a projection along the depth direction between the mouths of the openings arranged in the other strip-like element (10, 20); at least one channel (16, 26) extends in the depth direction over 50% or more of the total depth (T) of the drainage chamber (40); the opening surface area of the individual openings facing the drainage chamber (40) is 0.5mm² and preferably 1mm² or more, in a plane perpendicular to the depth direction; the opening surface area of the individual openings facing the drainage chamber (40) is 5mm² or less, preferably 4mm² or less, more preferably 3mm² or less in a plane perpendicular to the depth direction; the channel wall is arc-shaped, at least in sections, in a cutting plane parallel to the depth direction, and merges seamlessly into the boundary surface (12, 22); the distance between the inner boundary surfaces (14, 24) is 0.05mm, preferably 0.1mm or more, more preferably 0.3mm or more; and at least one strip-like element (10, 20) has a preferably perforated plastic film.

2. The wound covering according to claim 1, **characterised in that** the strip-like elements (10, 20) are connected to one another via point-shaped fixing sections (30), preferably designed in a grid arrangement.

3. The wound covering according to one of the preceding claims, **characterised in that** the individual fixing sections (30) have an area of 5mm² or less, preferably 3mm² or less, more preferably 2mm² or less in a cutting plane extending perpendicularly to the depth direction.

4. The wound covering according to Claim 2 or 3, **characterised in that** a material bridge connecting the inner boundary surfaces (14, 24) of the strip-like elements (10, 20) is formed in the fixing sections (30).

5. The wound covering according to one of the preceding claims, **characterised in that** at least one strip-like element (10, 20) comprises an antibacterial or bacteriostatic material, such as PHMB, silver or chlorhexidine, in particular in the form of a surface layer.

6. The wound covering according to one of the preceding claims, **characterised in that** at least one strip-like element comprises an agent, such as a hydrophilic or hydrophobic finish or the application of swellable materials, which counteracts the tendency of the wound covering to adhere.

7. Wound care kit with a wound covering according to one of Claims 1 to 6, an absorbent body of an occlusive film (50) applied thereon and an exudate line (6) passing through the occlusive film.

## Revendications

1. Pansement destiné à une utilisation dans le cadre d'une thérapie à pression négative pour des lésions de laparotomie, comprenant : un premier élément en forme de bande (10) formant une première surface de délimitation (12) et un second élément en forme de bande (20) relié au premier élément en forme de bande (10) et formant une deuxième surface de délimitation (22) qui est opposée et sensiblement parallèle à la première surface de délimitation (12) ; dans lequel il est formé au moins un espace de drainage (40) ouvert dépourvu de corps absorbant supplémentaire dans celui-ci, entre la première surface de délimitation intérieure (14) du premier élément en forme de bande (10), qui est opposée à la première surface de délimitation (12), et une deuxième surface de délimitation intérieure (24) du second élément en forme de bande (20), opposée à la deuxième surface de délimitation (22) ; dans lequel l'épaisseur (T) de l'espace de drainage, dans un sens de l'épaisseur perpendiculaire aux surfaces de délimitation (12, 22), est de 5 mm ou moins, de préférence de 4 mm ou moins et tout particulièrement de 2 mm ou moins afin d'assurer un effet capillaire sur les fluides corporels reçus dans l'espace de drainage (40), notamment les exsudats ; au moins un des éléments en forme de bande (10, 20) présente une ouverture permettant le passage des fluides corporels dans l'espace de drainage (40) ; il est formé au moins une ouverture dans un canal (16, 26) partant d'un élément en forme de bande (10, 20) en direction de la surface de délimitation intérieure (14, 24) opposée de l'autre élément en forme de bande (10, 20) et débouchant dans l'espace de drainage (40), ledit canal présentant une paroi réalisée de préférence d'un seul tenant avec l'élément en forme de bande (10, 20), notamment par perforation de l'élément en forme de bande (10, 20) ; la superficie transversale du canal (16, 26) va en rétrécissant dans un plan perpendiculaire au sens de l'épaisseur en partant de l'élément en forme de bande (10, 20) en direction de la surface de délimitation intérieure (14, 24) opposée, notamment afin d'obtenir un effet capillaire favorisant la pénétration des fluides corporels dans l'espace de drainage (40) ; au moins un élément en forme de bande (10, 20) présente une pluralité d'ouvertures agencées en ayant de préférence l'aspect d'une trame, l'écart entre des ouvertures adjacentes étant de 15 mm ou moins, de préférence de 5 mm ou moins, particulièrement de 3 mm ou moins ; les embouchures des ouvertures présentes dans un élément en forme de bande (10, 20) sont situées dans une projection le long du sens de l'épaisseur entre les embouchures des ouvertures présentes dans l'autre élément en forme de bande (10, 20) ; au moins un canal (16, 26) s'étend dans le sens de l'épaisseur sur 50 % ou plus de l'épaisseur (T) totale de l'espace de drainage (40) ; la superficie d'embouchure des diverses ouvertures, tournées vers l'espace de drainage (40), fait 0,5 mm² ou plus et de préférence 1 mm² ou plus dans un plan perpendiculaire au sens de l'épaisseur ; la superficie d'embouchure des diverses ouvertures tournées vers l'espace de drainage (40) fait 5 mm² ou moins, de préférence 4 mm² ou moins, tout particulièrement 3 mm² ou moins dans un plan perpendiculaire au sens de l'épaisseur ; la paroi de canal est de configuration arquée, au moins par sections, dans un plan de coupe parallèle au sens de l'épaisseur et est dans la continuité de ladite surface de délimitation (12, 22) ; l'écart entre les surfaces de délimitation intérieures (14, 24) est de 0,05 mm ou plus, de préférence de 0,1 mm ou plus, tout particulièrement de 0,3 mm ou plus ; et au moins un élément en forme de bande (10, 20) présente un film de plastique de préférence perforé.

2. Pansement selon la revendication 1, **caractérisé en ce que** les éléments en forme de bande (10, 20) sont fixés entre eux par des zones de fixations (30) en pointillé, en ayant de préférence l'aspect d'une trame.

3. Pansement selon l'une quelconque des revendications précédentes, **caractérisé en ce que** les diverses zones de fixation (30) présentent une surface de 5 mm² ou moins, de préférence de 3 mm² ou moins, tout particulièrement de 2 mm² ou moins dans un plan perpendiculaire au sens de l'épaisseur.

4. Pansement selon la revendication 2 ou 3, **caractérisé en ce qu'**un pont de matériau reliant les surfaces de délimitation intérieures (14, 24) des bandes de matériau (10, 20) est formé dans les zones de fixation (30).

5. Pansement selon l'une quelconque des revendications précédentes, **caractérisé en ce qu'**au moins un élément en forme de bande (10, 20) présente un matériau antibactérien ou bactériostatique, tel que le PHMB, l'argent ou la chlorhexidine, notamment sous la forme d'une couche superficielle.

6. Pansement selon l'une quelconque des revendications précédentes, **caractérisé en ce qu'**au moins un élément en forme de bande présente un moyen inhibant la tendance du pansement à l'adhérence, tel qu'un traitement hydrophile ou hydrophobe ou l'application de matériaux gonflants.

7. Trousse de traitement de lésion comportant un pansement selon l'une des revendications 1 à 6, un corps absorbant d'un film occlusif (50) appliqué sur ce dernier et un tuyau d'évacuation d'exsudat (6) traversant ledit film occlusif.
